Europäisches Patentamt

European Patent Office

Office européen des brevets

(19)

(11) Veröffentlichungsnummer: **0 395 896 B1**

(12) **EUROPÄISCHE PATENTSCHRIFT**

(45) Veröffentlichungstag der Patentschrift: **01.02.95**

(51) Int. Cl.6: **C07K 1/18**

(21) Anmeldenummer: **90106334.7**

(22) Anmeldetag: **03.04.90**

(54) **Verfahren zur Abtrennung von Toxinen aus Proteinlösungen.**

(30) Priorität: **10.04.89 DE 3911629**

(43) Veröffentlichungstag der Anmeldung:
**07.11.90 Patentblatt 90/45**

(45) Bekanntmachung des Hinweises auf die
Patenterteilung:
**01.02.95 Patentblatt 95/05**

(84) Benannte Vertragsstaaten:
**AT BE CH DE DK ES FR GB GR IT LI LU NL SE**

(56) Entgegenhaltungen:
**EP-A- 0 286 830**
**EP-A- 0 301 374**

(73) Patentinhaber: **BEHRINGWERKE Aktiengesellschaft**
**Postfach 1140**
**D-35001 Marburg (DE)**

(72) Erfinder: **Römisch, Jürgen, Dr.**
**Am Kähnelplatz 6**
**D-3550 Marburg (DE)**
Erfinder: **Heimburger, Norbert, Prof.**
**Sonnenhang 10**
**D-3550 Marburg (DE)**

EP 0 395 896 B1

**Beschreibung**

Die Erfindung betrifft ein Verfahren zur Abtrennung von Toxinen aus Lösungen von Proteinen, besonders von den Lipocortinen PP4, PP4-X (PAP II), PAP III, p68 sowie der Lipocortine I und II.

Das Plazenta-Gewebeprotein PP4, die Proteine PP4-X, PAP III und p68 sowie die Lipocortine I und II zeigen Homologie der Aminosäuresequenzen und gehören einer Familie von Proteinen an, die als Lipocortine bezeichnet werden.

Diese Proteine wirken entzündungshemmend und antikoagulatorisch. Sie wurden in vielen Organen nachgewiesen und können aus diesen isoliert werden.

Bei der Präparation aus Geweben, beispielsweise aus humaner Plazenta, oder von gentechnisch hergestellten, beispielsweise in E. coli exprimierten Proteinen, wie rPP4 oder rPP4-X, werden, zusammen mit diesen Proteinen, für den Menschen toxische Substanzen wie bakterielle Lipopolysacchsaride coisoliert. Trotz hoher Reinheit (größer als 95%, bzogen auf den Proteinanteil) zeigten die isolierten Proteine in Toxizitätsprüfungen wie dem Limulus-Test oder nach Applikation therapeutischer Dosen (1 mg Protein/kg Körpergewicht) an Kaninchen eine starke Verunreinigung mit toxischen Substanzen.

Diese offenbar Protein-assoziierten Verunreinigungen konnten weder durch chromatographische Verfahren oder Filtrationstechniken wie Sterilfiltration noch durch Einsatz nichtionischer Detergenzien oder von chelatbildenden Reagenzien allein oder in Kombination entfernt werden.

Aufgabe der Erfindung war es daher, Verfahren zur Entfernung von Toxinen stammend aus Organen, Geweben und Zellkulturen isolierter und auch gentechnisch hergestellter Proteine der Lipocortin-Familie zu entwickeln, die die biologische Aktivität der Proteine nicht schädigen und dadurch eine potentielle Verwendung als Gerinnungs-und/oder Entzündungstherapeutika ermöglichen.

Überraschenderweise wurde gefunden, daß in Anwesenheit chelatbildender Reagenzien in Kombination mit ionischen Detergenzien diese Proteine durch Ionenaustauschchromatographie von toxischen Substanzen befreit werden können, ohne daß die biologische Aktivität beeinträchtigt wird.

Gegenstand der Erfindung ist deshalb ein Verfahren zur Abtrennung von Toxinen aus einer Proteinlösung, dadurch gekennzeichnet, daß ein Protein in einer wässrigen Pufferlösung in Gegenwart eines Chelatbildners und eines ionischen Detergenzes einer Ionenaustauschchromatographie unter worfen wird.

Dieses Verfahren ist vor allem auf Lipocortine anwendbar, die natürlicher oder biotechnischer, besonders gentechnischer Herkunft sein können.

Als Chelatbildner kann beispielsweise EDTA, EGTA, ein Salz der Citronensäure oder Oxalsäure oder eine Kombination von diesen verwendet werden.

Als ionisches Detergenz kann beispielsweise Cholsäure, Taurocholsäure, Taurodehydrocholsäure, Desoxycholsäure, Taurodesoxycholsäure oder Taurochenodesoxycholsäure oder ein Salz von diesen oder ein Gemisch aus diesen verwendet werden.

Als Ionenaustauscher kann ein Anionenaustauscher bevorzugt DEAE-[R]Sepharose, -[R]Sephacel, -[R]Fractogel oder Q-[R]Sepharose, besonders bevorzugt DEAE-[R]Sepharose, verwendet werden.

Der Chelatbildner und das Detergenz können nach der erfindungsgemäßen Behandlung durch Dialyse oder durch Chromatographie in einer Pufferlösung mit pH 7,4-9,5, besonders bevorzugt pH 8,0-9,5, besonders bevorzugt pH 8,0-9,0, aus der proteinhaltigen Lösung entfernt werden.

In einer Verfahrensweise wird eine Lösung des Proteins, die eine Puffersubstanz, wie Tris, Glycin, Hepes oder PBS, mit einem pH-Wert von 7,0-10,0, und mindestens 0,1 mmol/l eines chelatbildenden Reagenzes, wie EDTA, EGTA, eines Salzes der Citronensäure oder der Oxalsäure oder einer Kombination von diesen und mindestens 0,05 g/l eines ionischen Detergenzes, wie Na-chol., Na-Doc., Na-Tdoc., Na-Tchol., Tcheno-Doc oder Tdcho. oder eines Gemisches aus diesen enthält, mit einem Anionenaustauscher in Kontakt gebracht, der Austauscher mit Pufferlösung gewaschen und das adsorbierte Protein mit einem Salz-Gradienten, z.B. mittels LiCl, KCl oder NaCl eluiert.

In einer bevorzugten Verfahrensweise wird eine Lösung des Proteins, mit einer Konzentration von 0,01-30 mg/ml, besonders bevorzugt 0,2-5 mg/ml, die Tris in einer Konzentration von 2-80 mmol/l und einem pH-Wert von 7,0-9,5, besonders bevorzugt 20 mmol/l Tris/HCl und einem pH-Wert von 8,0-9,0, enthält sowie 1-100 mmol/l eines chelatbildenden Reagenzes, besonders bevorzugt 5-20 mmol/l EDTA, und 0,2-5 g/l, besonders bevorzugt 0,8-1,5 g/l Na-chol. oder Na-Doc. oder eines Gemisches, mit DEAE-[R]Sepharose, -[R]Sephacel, -[R]Fractogel, oder Q-[R]Sepharose, besonders bevorzugt DEAE-[R]Sepharose, in Kontakt gebracht. Nach Waschen des Austauschers mit Pufferlösung wird das adsorbierte Protein mit einem linearen steigenden NaCl-Gradienten eluiert.

Die protein-haltigen Säulendurchläufe oder -Eluate können von chelatbildenden Reagenzien und Detergenzien durch Dialyse gegen eine Pufferlösung aus Tris, Hepes, Glycin oder PBS, besonders bevorzugt gegen eine Pufferlösung mit pH 8,0-9,0, oder durch einen weiteren chromatographischen Schritt wie

2

Gelpermeationschromatographie mit AcA 202 oder AcA 54 befreit werden.

Gegebenenfalls können die so behandelten Präparationen weiter gereinigt werden.

Für die Beschreibung wurden folgende Abkürzungen verwendet:

DEAE:              Diethylaminoethyl
EDTA:              Ethylendiamintetraessigsäure
Hepes:             N-2-Hydroxyethylpiperazin-N-2-Ethansulfonsäure
Na-Chol:           Natrium-Cholat
Na-Doc:            Natrium-Desoxycholat
Na-Tchol:          Natrium-Taurocholat
Na-Tdoc:           Natrium-Taurodesoxycholat
PBS:               Natrium- oder Kalium-Phosphat-Puffer
rPP4:              gentechnologisch hergestelltes, in E. coli exprimiertes PP4
rPP4-X:            gentochnologisch hergestelltes, in E. coli exprimiertes PP4-4
PAP III:           plazentares antikoagulatorisches Protein III
PAGE:              Polyacrylamid-Gelelektrophorese
Q:                 Quarternär-Amin
SDS:               Natriumdodecylsulfat
Tcheno-Doc:        Taurochenodesoxycholsäure
Tdchol:            Taurodehydrocholsäure
Tris:              Tris(hydroxymethyl)aminomethan

Die Erfindung wird an nachstehenden Beispielen erläutert:

Als Ausgangsubstanzen für die Detoxifizierung wurden Präparationen der Proteine PP4, PP4-X, PAP III, p68, Lipocortine I und II aus humaner Plazenta und der Proteine rPP4 und rPP4-X aus transformierten E. coli-Kulturen mit einer Reinheit, bezogen auf den Proteinanteil, von größer als 95%, in einer Pufferlösung aus 0,02 mol/l Tris/HCl, pH 8,5, mit einer Proteinkonzentration von 2,5 mg/ml eingesetzt. Diese Präparationen wiesen einen deutlichen Gehalt an toxischen Substanzen auf (Tabelle I) wie mit Hilfe des Limulus-Testes (durchgeführt in Lösungen bei pH 7,2) und des Tiermodells bestimmt wurde.

Toxizitätstests

1. Limulus-Test

Dieser Test wurde wie von der Firma "Concept GmbH (Heidelberg, Deutschland) beschrieben durchgeführt: 0,1 ml der zu prüfenden protein-haltigen Lösung wurde mit 0,1 ml Limulus-Amoebozyten-Lysat in einem pyrogenfreien Röhrchen sanft vermischt und das Röhrchen erschütterungsfrei während 60 min bei 37°C inkubiert. Nach dem Ende der Inkubationzeit wurde das Röhrchen darauf visuell geprüft, ob sich ein festes Gel gebildet hatte.

Die Pyrogenität der getesteten Substanz, ausgedrückt in EU (Endotoxin Units), wurde mittels einer Eichkurve ermittelt, die mit Hilfe eines Referenz-Endotoxins (EC-5) erstellt wurde.

2. Pyrogen-Test am Kaninchen:

Die Toxizität der Proteinproben wurde durch Messung der Erhöhung der Körpertemperaturen (rectal) von Kaninchen gegenüber der Körpertemperatur, die in einem 90minütigen Vortest bestimmt wurde, bestimmt. Proteinproben wurden den Kaninchen i.v. in eine Ohrvene im Bolus appliziert (1 mg/kg Körpergewicht) und die Körpertemperaturen über einen Zeitraum von 180 min registriert. Der höchste Wert wurde für die Auswertung zugrunde gelegt. Als pyrogenfrei wurden Proben dann bewertet, wenn die Summe der Temperaturdifferenzen von 6 Tieren kleiner oder gleich 2,2°C war.

Beispiel 1

Nach Zugabe von EDTA auf eine Endkonzentration von 0,01 mol/l, unter Kontrolle des pH-Wertes, und 0,1% Na-Doc wurden die PP4-, rPP4-, PP4-X-, rPP4-X-, PAP III-, p68- oder Lipocortin I- oder II-haltigen Lösungen mit DEAE-$^R$Sepharose (Fa. Pharmacia, Schweden), äquilibiert mit 0,02 M Tris/HCl, pH 8,5, 0,01 M EDTA und 0,1% Na-Doc. (Säulenpuffer) in einer Säule in Kontakt gebracht, das Gelmaterial mit Säulenpuffer gewaschen und adsorbierte Proteine mit einem linear steigenden NaCl-Gradienten eluiert.

Die Eluate wurden extensiv gegen eine Pufferlösung aus 0,02 mol/l Tris/HCl, pH 8,5, und anschließend gegen eine Pufferlösung aus 0,02 mol/l Tris/HCl, pH 7,2, dialysiert und die Dialysate sowohl im Limulus-Test als auch im Pyrogentest an Kaninchen auf Toxizität überprüft.

Die auf diese Weise behandelten Proteine riefen keine oder nur sehr geringe Temperaturerhöhungen im Pyrogentest oder kaum meßbare Endotoxingehalte im Limulus-Test hervor (Tabelle I) und konnten als pyrogenfrei bewertet werden.

Die Proteine PP4-X und rPP4-X wurden unter den genannten Bedingungen nicht an das Gelmaterial adsorbiert, sondern befanden sich im Säulendurchlauf, waren aber nach Durchführung des angegebenen Verfahrens ebenfalls pyrogenfrei (Tabelle I).

Die biologische Aktivität, überprüft mit Hilfe der modifizierten Thromboplastinzeit, bezogen auf die Proteinkonzentration, blieb durch diesen Verfahrensschritt voll erhalten gegenüber den Ausgangsmaterialien.

Die Ausbeuten der Proteine lagen zwischen 64 und 85 %, bezogen auf die toxischen Ausgangsmaterialien.

Beispiel 2

PP4-, rPP4-, PP4-X-, rPP4-X-, PAP III-, p68- oder Lipocortin I- oder II-haltige Pufferlösungen wurden Na-Chol oder Na-Tchol bis zu einer Endkonzentration von jeweils 0,5 g/l beigemischt sowie 0,01 mol/l EDTA, diese mit Q-$^R$Sepharose (Fa. Pharmacia, Schweden) äquilibriert, mit 0,02 mol/l PBS, pH 8,5, 0,01 mol/l EDTA, 0,05 g/l Na-Chol und 0,5 g/l Na-Tchol (Säulenpuffer) in einer Säule in Kontakt gebracht, das Gelmaterial gewaschen und adsorbierte Proteine mit einem linear steigenden NaCl-Gradienten eluiert.

Zur weiteren Behandlung der Proteinlösungen und deren Überprüfung auf Toxizität wurde wie in Beispiel 1 beschrieben verfahren. Die Ergebnisse dieser Untersuchungen entsprachen denen für Beispiel 1 und sind in Tabelle I aufgeführt.

Tabelle I

| Protein | Ausgangsmaterial | | nach Detoxifizierung | | | Ausbeute[3] |
| | Toxizitätstest | | Toxizitätstest | | Gerinnungs- | |
| | Limulus | Kaninchen[1] | Limulus | Kaninchen | inhibition[2] | |
| | EU/ml | t(°C) | EU/ml | t(°C) | % | % |
| --- | --- | --- | --- | --- | --- | --- |
| PP4 | * 250 | 6,6 | ** 0,25 | 0,8 | 100 | 70 |
| rPP4 | * 250 | 10,8 | 2,0 | 1,4 | 100 | 65 |
| PP4-X | 50 | 4,8 | ** 0,25 | 1,0 | 96 | 80 |
| r-PP4-X | * 250 | 9,0 | 2,0 | 1,2 | 95 | 70 |
| p68 | 250 | 6,0 | 0,5 | 0,8 | 97 | 65 |
| Lipocortin I | 50 | 5,4 | 0,5 | 1,4 | 93 | 70 |
| Lipocortin II | 50 | 4,8 | 0,25 | 1,0 | 98 | 85 |
| Faktor XIII | 50 | 3,6 | 50 | 3,4 | - | 80 |

\* größer als

** kleiner als

1 Temperaturdifferenzen (siehe Text); Summe aus 6 Kaninchen

2 bezogen auf die Gerinnungsinhibition (modifizierter Thromboplastintest) der Ausgangsmaterialien (= 100 %)

3 bezogen auf die zur Detoxifizierung eingesetzte Menge (= 100 %) an Reinstproteinen

**Patentansprüche**

1. Verfahren zur Abtrennung von Toxinen aus Proteinlösungen, dadurch gekennzeichnet, daß ein Protein in einer wässrigen Pufferlösung in Gegenwart eines Chelatbildners und eines ionischen Detergenzes einer Ionenaustauscherchromatographie unterworfen wird.

2. Verfahren nach Anspruch 1, dadurch gekennzeichnet, daß das Protein ein Lipocortin ist.

3. Verfahren nach Anspruch 1, dadurch gekennzeichnet, daß der Chelatbildner EDTA, EGTA, ein Salz der Citronensäure oder Oxalsäure oder eine Kombination von diesen verwendet wird.

4. Verfahren nach Anspruch 3, dadurch gekennzeichnet, daß der Chelatbildner in einer Konzentration von 1-100 mmol/l verwendet wird.

5. Verfahren nach Anspruch 1, dadurch gekennzeichnet, daß als Detergenz Cholsäure, Taurocholsäure, Taurodehydrocholsäure, Desoxycholsäure, Taurodesoxycholsäure oder Taurochenodesoxycholsäure oder ein Salz von diesen oder ein Gemisch aus diesen verwendet wird.

6. Verfahren nach Anspruch 5, dadurch gekennzeichnet, daß das Detergenz in einer Konzentration von 0,02-5 g/l verwendet wird.

7. Verfahren nach Anspruch 5, dadurch gekennzeichnet, daß als Ionenaustauscher DEAE-[R]Sepharose, -[R]Sephacel, -[R]Fractogel oder Q-[R]Sepharose verwendet wird.

8. Verfahren nach Anspruch 1, dadurch gekennzeichnet, daß als Ionenaustauscher DEAE-[R]Sepharose verwendet wird.

9. Verfahren nach Anspruch 1, dadurch gekennzeichnet, daß Chelatbildner und Detergenz durch Dialyse oder Chromatographie in einer Pufferlösung mit pH 7,5-9,5 aus der proteinhaltigen Lösung entfernt werden.

**Claims**

1. A process for removing toxins from protein solutions, which comprises subjecting a protein in an aqueous buffer solution in the presence of a chelating agent and of an ionic detergent to an ion exchange chromatography.

2. The process as claimed in claim 1, wherein the protein is a lipocortin.

3. The process as claimed in claim 1, wherein EDTA, EGTA, a salt of citric acid or oxalic acid or a combination of the latter is used as chelating agent.

4. The process as claimed in claim 3, wherein the chelating agent is used in a concentration of 1-100 mmol/l.

5. The process as claimed in claim 1, wherein cholic acid, taurocholic acid, taurodehydrocholic acid, deoxycholic acid, taurodeoxycholic acid or taurochenodeoxycholic acid or a salt of the latter or a mixture of the latter is used as detergent.

6. The process as claimed in claim 5, wherein the detergent is used in a concentration of 0.02-5 g/l.

7. The process as claimed in claim 5, wherein DEAE-[R]Sepharose, -[R]Sephacel, -[R]Factogel or Q-[R]Sepharose is used as ion exchanger.

8. The process as claimed in claim 1, wherein DEAE-[R]Sepharose is used as ion exchanger.

9. The process as claimed in claim 1, wherein the chelating agent and detergent are removed from the protein-containing solution by dialysis or chromatography in a buffer solution of pH 7.5-9.5.

**Revendications**

1. Procédé pour la séparation de toxines d'avec des solutions de protéines, caractérisé en ce que l'on soumet une protéine à une chromatographie d'échange d'ions, dans une solution aqueuse tampon, en présence d'un agent chélateur et d'un détergent ionique.

2. Procédé selon la revendication 1, caractérisé en ce que la protéine est une lipocortine.

3. Procédé selon la revendication 1, caractérisé en ce que, comme agent chélateur, on utilise l'EDTA, l'EGTA, un sel de l'acide citrique ou oxalique, ou un mélange de ceux-ci.

4. Procédé selon la revendication 1, caractérisé en ce que l'on utilise l'agent chélateur à une concentration de 1-100 mmoles/l.

5. Procédé selon la revendication 1, caractérisé en ce que, comme détergent, on utilise l'acide cholique, taurocholique, taurodéhydrocholique, désoxycholique, taurodésoxycholique ou taurochénodésoxycholique, ou un sel de ceux-ci ou un mélange de ceux-ci.

6. Procédé selon la revendication 5, caractérisé en ce que l'on utilise le détergent à une concentration de 0,02-5 g/l.

7. Procédé selon la revendication 5, caractérisé en ce que, comme échangeur d'ions, on utilise le DEAE-Sepharose®, -Sephacel®, -Fractogel® ou Q-Sepharose®.

8. Procédé selon la revendication 1, caractérisé en ce que, comme échangeur d'ions, on utilise le DEAE-Sepharose®.

9. Procédé selon la revendication 1, caractérisé en ce que l'agent chélateur et le détergent sont éliminés de la solution contenant la protéine par dialyse ou chromatographie dans une solution tampon à pH 7,5-9,5.